# EUROPEAN PATENT SPECIFICATION

(11) **EP 0 470 452 B1**
(45) Date of publication and mention of the grant of the patent: **11.10.1995**
(21) Application number: 91112558.1
(22) Date of filing: 26.07.1991
(51) Int. Cl.: C08B 37/16, A61K 47/40

(54) **A method for the production of complexes of long chain polyunsaturated fatty acids and their derivatives, with cyclodextrins, and the resulting complexes**
Verfahren zur Herstellung von Komplexen aus mehrfachungesättigten Fettsäuren mit langer Kette und Cyclodextrinen und die so erhaltenen Komplexe
Procédé de préparation de complexes d'acides gras polyinsaturés à longue chaîne avec des cyclodextrines

(30) Priority: 09.08.1990 IT 2125790
(43) Date of publication of application: 12.02.1992
(73) Proprietor: STAROIL LIMITED, British Virgin Islands (GB)
(72) Inventor: Bruzzese, Tiberio, I-20146 Milano (IT); Mozzi, Giovanni, I-20131 Milan (IT)
(74) Representative: Luksch, Giorgio, Dr.-Ing.

(56) References cited:
- FR-A- 2 550 445
- FR-A- 2 596 617
- US-A- 2 827 452

## Description

The present invention is concerned with both a method of producing new complexes of long chain polyunsaturated fatty acids, their salts and esters, inclusive of fish and vegetable oil glycerides, with cyclodextrins (α-, β-and hydroxypropyl-β-cyclodextrin), and the resulting new complexes.
The dietetic and pharmaceutical use of fish oils and of polyunsaturated fatty acids, therein contained in the form of glycerides, is extending on the ground of their claimed fat lowering (Y. Tamura et al., Prog. Lipid. Res., 25, 461, 1986; C. Von Schacky, Ann. Intern. Med., 107, 890, 1987) and platelet anticoagulant properties (C.R.M. Hay et al., Lancet, ii, 1269, 1982), highly interesting in the prevention and treatment of the most important cardiovascular diseases. At the same time a most pressing evidence of other therapeutic uses is arising in the treatment of psoriasis, rheumatoid arthritis (J.M. Kremer et al., Ann. Intern. Med., 106, 497, 1987; R.T. Sperling et al., Arth. Rheum., 30, 988, 1987), polyposis of the colon , arterial hypertension (K.M. Bonaa et al., N. Engl. J. Med., 322, 795, 1990; M.R. Knapp et al., N. Engl. J. Med., 320, 1037, 1989) and in the therapy of cancer.

The fish oils, some vegetable oils, the polyunsaturated fatty acids making them up and their derivatives exhibit however some characteristics markedly limiting their use. They are liquid at room temperature, unctuous, with an unpleasant taste and odour and easily oxidized in the air owing to the large number of carbon-carbon double bonds in their molecules, with consequent deterioration in time of the organoleptic characteristics and potential formation of epoxy groups considered as having toxic effects.
These characteristics markedly curtail the dietetic and pharmaceutical use of these products. Dosage forms such as tablets are for instance precluded as are hard gelatin capsules, syrups, drinking vials, water-dispersible granules in single dose sachets.
In practice the sole pharmaceutical form used is the soft gelatin capsules, normally of considerable size owing to the rather high dosage (up to several grams daily). This extra large size makes the use of the capsules by the elderly quite difficult, who are the principal users owing to the pathologies mentioned above and who would have no difficulty if they were to take e.g., syrups or water dispersible granules.
To forestall these disadvantages U.S. Patent no. 4 438 106 describes inclusion compounds of eicosapentaenoic acid (EPA) and docosahexaenoic acid (DHA), their alkaline salts and alkyl esters in cyclodextrin, obtained by a complexation reaction occurring in the presence of large quantities of a polar organic solvent and through boiling. In the resulting complexes the quantity of oleaginous substance is not more than 15% by weight at the best of times. A like concentration of active substance requires that frequent daily doses be administered in order to attain the pharmacological active quantity of active ingredient; moreover oversize dosage units will have to be prepared to ensure such quantities, not to mention the excessive use of the complexant cyclodextrin, inactive per se but with a negative influence as regards the cost of the finished product. Moreover, the known process calls for the use of large quantities of solvents negatively influencing the production costs and entailing rather considerable risks to the operator (fire, explosion, intoxication by inhalation) and to the user (unavoidable traces of solvents in the finished product).

It was surprisingly discovered that a process requiring no organic solvents permitted the production of complexes of long chain polyunsaturated fatty acids, their salts and esters, inclusive of fish and vegetable oil glycerides, with cyclodextrins ( α -, β- and hydroxypropyl-β-cyclodextrin) having a content by weight of oleaginous substance significantly higher than the limits of the known art as mentioned above, with consequent avoidance of the before mentioned disadvantages.
The method of the invention comprises: solution of cyclodextrin in simple, preferably distilled, water, introduction of the active oleaginous substance in the resulting solution thereby obtaining an heterogenic mixture, submission of the mixture to stirring for from about 1 to about 24 hours at a temperature comprised between 0 and 100°C, preferably at room temperature, from which the desired complex precipitates in a solid, crystalline form; the complex is recovered by filtration thereafter washed and dried. Specifically, the present invention relates to a method of producing a complex containing at least one compound selected from the group consisting of eicosapentaenoic acid (EPA) and docosahexaenoic acid (DHA), a salt thereof or a C₁-C₃ alkyl or glyceryl ester thereof and an α-, β- or β-hydroxypropyl cyclodextrin characterized in that said cyclodextrin is dissolved in water, said compound or the derivative thereof is added at room temperature to said aqueous solution to form a heterogeneous mixture which is submitted for a period of 1 to 24 hours to stirring and wherefrom the complex precipitates in crystalline solid form and recovered according to known techniques.

The phase of washing the invention complex is carried out with water or with organic solvent such as methanol, ethanol, etc., or their mixture. Drying may be done in oven at a moderate temperature.
FR-A-2 550 445 discloses a method wherein a cyclodextrin and a fatty acid or a suitable derivative thereof are slurried among themselves in the presence of small volumes of water and working at relatively high temperatures. Said method allows to obtain high inclusion percentages of the fatty acid only with γ-cyclodextrins, thus demonstrating that it is arbitrary to extend a method which is valid for one class of cyclodextrins to all classes.
FR-A-2 596 617 discloses a method which provides to dissolve β-cyclodextrins in water at high temperatures, and to add a fatty acid or an oxidation derivative thereof in the hot solution (80°C), in order to carry out the reaction at this temperature. No attempt is made either to determine the acid content of the complex, nor to improve it by optimizing the process conditions. No example is therein referred to the possibility to use the other classes of cyclodextrins, therefore, also in view of the above arguments as to FR-A-2 550 445 the extrapolation to whichever class of cyclodextrins appears arbitrary.

Also US 2 827 452 claims a method for preparing inclusion complexes made of organic materials and amides or dextrins, among which there are disclosed cyclodextrins.
The complexes attained show an inclusion percentage of the organic material which ranges between 7 and 12; the purpose is to stabilize unstable materials, especially as to oxidization; no mention is made to solid pharmaceutical forms different from soft gelatin capsules, odorless and without unpleasant tastes.

The resulting products are characterized by the fact that the concentration of oleaginous substance present in the complex is higher than 18% by weight, preferably from about 20% to about 50% by weight.
Starting from the acids themselves analogous complexes were prepared in a free or variously salified form, or from the corresponding C₁-C₃ esters (preferably from ethyl esters), or from the relative glycerides obtained through synthesis.
All the above mentioned compounds can be present in the oils used in the complexation, either alone, as a single component, or as a mixture of them in a varying ratio.
The cyclodextrins that can be used in complexation may be either α-, β- or cyclodextrin, characterized by 6, 7 or 8 D-glucopyranoside units forming a cyclic cavity. Hydroxypropyl-β-cyclodextrin can also be used; it is derived from β-cyclodextrin through total or partial replacement of the hydroxyls of the ring-forming glucoside units with an -O-CH₂-CHOH-CH₃ radical or one of its polymers

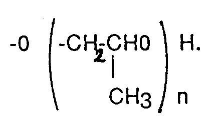

Unlike the oleaginous substances used in the preparation, the new complexes appear as gliding, not unctuous, nearly odourless and tasteless powders and can therefore be used to prepare a variety of pharmaceutical forms such as tablets, hard gelatin capsules , syrups, drinking vials, water dispersible granules in sachets etc. Furthermore, they maintain in time their satisfactory initial organoleptic and chemical characteristics, since their stability is better than the starting oils.

In this context the term 'complexes' specially means inclusion compounds of long chain polyunsaturated substances enclosed in the hydrophobic cavities of the cyclodextrins.

The following examples are solely given to better illustrate the invention, but in no way are they to be intended as limiting the scopes of the invention itself.

The oil present in the complex was determined by performing the U.V. absorption spectrum at 195 to 300 nm of an ethanol solution of it and then comparing the maximum absorbance with that of an analogous standard solution.

The gas-chromatographic determination (G.C.) of the oil present in the complex was performed on a chloroform extract of it under the following operative conditions:
- internal standard: 0.003% methyl palmitate
- column : SBT ^{TB}-1, 15 m, 0.25 mm int. dia.,
   0.25 µ thickness
- Injection port: PTV
- splitting: 1:70
- column temp. programmed at from 160 to 240°C
- carrier gas: helium
- adjuvant gas: nitrogen
- detector: F.I.D.

The yields were obtained by calculating the percent ratio between product obtained and sum of oil plus starting cyclodextrin.

The cyclodextrins used contained varying quantities of water (up to 12%). In the following examples the percent assay values of the cyclodextrins used are shown in brackets.

### Example 1

Dissolve 18.1 g of β-cyclodextrin (assay value about 88% calculated on the substance as such) in 500 ml of distilled water at 40-45°C. Cool to room temperature and while stirring add 5 g of oleaginous substance containing DHA ethylester (assay value ≧ 80%). Cool immediately to 2°C and maintain under stirring at this temperature for 7 h. Filter, wash with about 50 ml of distilled water and dry in oven at 45°C.
The product obtained with a yield of about 85%, contains 23.7% of oil (G.C. assay value on the dry product) equivalent to a molar ratio to β-cyclodextrin of about 1:1.

### Example 2

Dissolve 18.1 g of β-cyclodextrin (assay value about 88% calculated on the substance as such) in 500 ml of distilled water at 40-45°C. Cool to room temperature and add 10 g of oleaginous substance containing DHA ethylester (assay value ≧80%). Agitate on shaker for 22 h at 26°C. Filter, wash with about 50 ml of distilled water and dry in an oven at 45°C.
The product, obtained with a yield of about 93%, contains 38.5% of oil (G.C. assay value on the dry product) equivalent to a molar ratio to β-cyclodextrin of about 2 : 1.

### Example 3

Dissolve 38 g of β-cyclodextrin (assay value about 88% calculated on the substance as such) in 1 litre of distilled water at 40-45°C. Cool to room temperature and while stirring add 20 g of oleaginous substance containing EPA and DHA ethylesters (combined assay value ≧ 80%). Maintain under stirring at room temperature for 7 h. Filter, wash with about 100 ml of distilled water and dry in an oven at 45°C.
The product, obtained with a yield of about 90%, contains 36.6% of oil (G.C. assay value on the dry product), equivalent to a molar ratio to β-cyclodextrin of about 2:1.

### Example 4

Dissolve 19 g of β-cyclodextrin (assay value about 88% calculated on the substance as such) in 500 ml of distilled water at 40-45°C. Cool to room temperature and while stirring add 17.3 g of oleaginous substance containing EPA and DHA ethylesters (combined assay value ≧ 80%). Maintain under stirring at room temperature for 7 h. Filter, wash with about 50 ml of distilled water and dry in an oven at 45°C.
The product, obtained with a yield of about 80%, contains 44.4% of oil (G.C. assay value on the dry product), equivalent to a molar ratio to β-cyclodextrin of about 3:1.

### Example 5

Dissolve 3.2 g of α-cyclodextrin (assay value about 90% calculated on the substance as such) in 20 ml of distilled water and while stirring add 1 g of oleaginous substance containing EPA and DHA ethylesters (combined assay value ≧ 80%). Maintain under stirring at room temperature for 5 h. Filter, wash with about 10 ml of distilled water and dry in an oven at 45°C.
The product, obtained with a yield of about 96%, contains 26.3% of oil (G.C. assay value on the dry product), equivalent to a molar ratio to α-cyclodextrin of about 1:1.

### Example 6

Dissolve 3.9 g of β-cyclodextrin (assay value about 88% calculated on the substance as such) in 100 ml of distilled water at 40-45°C. Cool to room temperature and while stirring add 1 g of oleaginous substance containing EPA and DHA triglyceride esters (combined assay value > 50%). Maintain under stirring at room temperature for 4.5 h. Filter, wash with about 20 ml of distilled water and dry in an oven at 45°C.
The product, obtained with a yield of about 80%, contains 22.2% of oil (U.V. assay value on the dry product), equivalent to a molar ratio of the triglyceride acid radical to β-cyclodextrin of about 1:1.

### Example 7

Dissolve 3.4 g of β-cyclodextrin (assay value about 88% calculated on the substance as such) in 50 ml of distilled water at 40-45°C. Cool to room temperature and dissolve 1 g of DHA sodium salt. Maintain under stirring at room temperature for 3 h and evaporate the solution to dryness under reduced pressure then wash the residue with 5 ml of distilled water, filter and dry in an oven at 45°C.
The product obtained contains 20.6% of DHA sodium salt (U.V. assay value on the dry product), equivalent to a molar ratio to β-cyclodextrin of about 1:1.25.

### Example 8

Dissolve 4.2 g of β-cyclodextrin (assay value about 88% calculated on the substance as such) in 100 ml of distilled water at 40-45°C. Cool to room temperature and while stirring add 1 g of an oleaginous substance containing EPA acid (assay value ≧ 80%). Maintain under stirring at room temperature for 5.5 h. Filter, wash with about 20 ml of distilled water and dry in an oven at 45°C.
The product obtained contains 19.6% of oil (U.V. assay value on the dry product), equivalent to a molar ratio to β-cyclodextrin of about 1:1.

### Example 9

Dissolve 3.9 g of β-cyclodextrin (assay value about 88% calculated on the substance as such) in 100 ml of distilled water at 40-45°C. Cool to room temperature and add 2 g of oleaginous substance containing EPA ethylester (assay value ≧ 80%). Agitate on a shaker for 22 h at 26°C. Filter, wash with about 20 ml of distilled water and dry in an oven at 45°C.
The product, obtained with a yield of about 90%, contains 35.7% of oil (U.V. assay value on the dry product), equivalent to a molar ratio to β-cyclodextrin of about 2:1.

## Claims

1. A method of producing a complex containing at least one compound selected from the group consisting of eicosapentaenoic acid (EPA) and docosahexaenoic acid (DHA), a salt thereof or a C₁-C₃ alkyl or glyceryl ester thereof and an α-, β- or hydroxypropyl-β-cyclodextrin characterized in that said cyclodextrin is dissolved in water, said compound or the derivative thereof is added at room temperature to said aqueous solution to form a heterogeneous mixture which is submitted for a period of 1 to 24 hours to stirring at a temperature of between 0° and 100°C and wherefrom the complex precipitates in crystalline solid form and recovered by filtration, washing and drying.

2. A method in accordance with the foregoing claim characterized in that said compound is selected from the group consisting of eicosapentaenoic acid (EPA) and docosahexaenoic acid (DHA).

3. A method in accordance with claim 1, characterized in that said alkyl ester derivatives are ethylesters.

4. A method in accordance with the foregoing claims characterized in that the alkyl ester is EPA ethylester and the cyclodextrin is a β-cyclodextrin.

5. A method in accordance with claims 1-4 characterized in that the alkyl ester is DHA ethylester and the cyclodextrin is a β-cyclodextrin.

6. A method in accordance with claims 1-4 characterized in that the alkyl ester is EPA ethylester+DHA ethylester and the cyclodextrin is a β-cyclodextrin.

7. A complex produced by the method according to any of the foregoing claims.

## Patentansprüche

1. Verfahren zur Herstellung von Komplexen, bestehend aus mindestens einer Verbindung aus der Gruppe der Eicosapentansäure (EPA) und Docosahexansäure (DHA), einem ihrer Salze oder einem ihrer C₁-C₃-Alkyl- oder Glycerinester und einem α-, β- oder Hydroxypropyl-β-cyclodextrin; **dadurch gekennzeichnet**, daß das besagte Cyclodextrin in Wasser aufgelöst wird, besagte Verbindung oder eines ihrer Derivate bei Umgebungstemperatur der besagten wäßrigen Lösung zugesetzt und so eine homogene Mischung gebildet wird, die über einen Zeitraum zwischen 1 und 24 Stunden bei einer Temperatur zwischen 0° und 100°C umgerührt wird, und aus der der Komplex in kristalliner fester Form ausgefällt und durch Filtern, Waschen und Trocknen erhalten wird.

2. Verfahren nach Anspruch 1, **dadurch gekennzeichnet**, daß besagte Verbindung aus der Gruppe der Eicosapentansäure (EPA) und Docosahexansäure (DHA) ausgewählt wird.

3. Verfahren nach Anspruch 1, **dadurch gekennzeichnet**, daß es sich bei den besagten Alkylesterderivaten um Ethylester handelt.

4. Verfahren nach einem der vorhergehenden Ansprüche, **dadurch gekennzeichnet**, daß es sich bei dem Alkylester um EPA-Alkylester und bei dem Cyclodextrin um ein β-Cyclodextrin handelt.

5. Verfahren nach den Ansprüchen 1 bis 4, **dadurch gekennzeichnet**, daß es sich bei dem Alkylester um DHA-Ethylester und bei dem Cyclodextrin um ein β-Cyclodextrin handelt.

6. Verfahren nach den Ansprüchen 1-4, **dadurch gekennzeichnet**, daß es sich bei dem Alkylester um EPA-Ethylester + DHA-Ethylester und bei dem Cyclodextrin um ein β-Cyclodextrin handelt.

7. Komplex, hergestellt mit dem Verfahren nach einem beliebigen der vorangegangenen Ansprüche.

## Revendications

1. Procédé de préparation d'un complexe contenant au moins un composé choisi dans le groupe constitué par de l'acide eicosapentaénoïque (EPA) et de l'acide docosahexaénoïque (DHA), un sel de celui-ci ou un alkyle C₁-C₃ ou un ester glycéryle de celui-ci, et une α-cyclodextrine, β-cyclodextrine ou hydroxypropyle-β-cyclodextrine, caractérisé en ce que ladite cyclodextrine est dissoute dans l'eau, ledit composé ou son dérivé est ajouté à la température de la pièce à ladite solution aqueuse pour former un mélange hétérogène qui est placé dans un agitateur pendant une durée de 1 à 24 heures à une température comprise entre 0° et 100°C, à la suite de quoi le complexe précipite sous forme d'un solide cristallin et est récupéré par filtration, lavage et séchage.

2. Procédé selon la revendication précédente, caractérisé en ce que ledit composé est choisi dans le groupe constitué par de l'acide eicosapentaénoïque (EPA) et de l'acide docosahexaénoïque (DHA).

3. Procédé selon la revendication 1, caractérisé en ce que lesdits dérivés d'ester d'alkyle sont des éthylesters.

4. Procédé selon les revendications précédentes, caractérisé en ce que l'ester d'alkyle est un éthylester d'EPA et la cyclodextrine est une β-cyclodextrine.

5. Procédé selon les revendications 1 à 4, caractérisé en ce que l'ester d'alkyle est un éthylester de DHA et la cyclodextrine est une β-cyclodextrine.

6. Procédé selon les revendications 1 à 4, caractérisé en ce que l'ester d'alkyle est un éthylester d'EPA + un éthylester de DHA et la cyclodextrine est une β-cyclodextrine.

7. Complexe préparé par le procédé selon l'une quelconque des revendications précédentes.
